# EUROPEAN PATENT APPLICATION

(11) **EP 4 470 607 A1**
(43) Date of publication of application: **04.12.2024**
(21) Application number: 23746916.8
(22) Date of filing: 24.01.2023
(51) Int. Cl.: A61N 2/04

(54) **MAGNETIC THERAPY DEVICE**

(30) Priority: 25.01.2022 JP 2022009369
(71) Applicant: Nipro Corporation, Settsu-shi, Osaka 566-8510 (JP); Harada Electronics Industry Co., Ltd., Sapporo-shi, Hokkaido 063-0052 (JP)
(72) Inventor: NAKAZAWA Koji, Chuo-shi, Yamanashi 409-3801 (JP); MURAKAMI Masaki, Chuo-shi, Yamanashi 409-3801 (JP); HARADA Masahide, Sapporo-shi, Hokkaido 063-0052 (JP)
(74) Representative: Mewburn Ellis LLP
(86) International application number: PCT/JP2023/002004
(87) International publication number: WO 2023/145705

(57) **Abstract**

Provided is a magnetic treatment apparatus with which there can be achieved a therapeutic effect required to treat pain in an affected area by increasing the output of a high-frequency signal, and with which the safety of the apparatus itself can be ensured as well. The magnetic treatment apparatus is used to treat pain in an affected area, and includes: an apparatus main body having a signal wave output part configured to generate and output a biostimulation signal wave of a first frequency; and a probe formed separately from the apparatus main body and having a first coil that is connected to the signal wave output part through a signal cable and is supplied with the biostimulation signal wave of the first frequency outputted from the signal wave output part, wherein the first coil is formed as a planar body on one or both surfaces of a flexible sheet, and a coil for magnetic field intensity detection that is adjacent to the first coil is formed on one or both surfaces of the flexible sheet.

## Description

### Technical Field

The present invention relates to a treatment apparatus for treating pain in an affected area of a living body by generating a biostimulation signal wave and stimulating the cells in the affected area as a result of irradiating the affected area with a magnetic field that is generated at a coil via such signal wave.

### Background Art

As an apparatus for treating pain in an affected area of a living body by stimulating the cells in the affected area as a result of irradiating the affected area with a magnetic field, there is conventionally known, for example, an apparatus disclosed in Patent Literature 1. This treatment apparatus is configured in such a manner that a helical high-frequency coil and a helical low-frequency coil are aligned together in a housing, or a looped high-frequency coil and a looped low-frequency coil are stacked together in a housing, whereby these coils are received in the housing along with an oscillation circuit and a battery, which allows the apparatus to be carried around.

Further, this treatment apparatus treats pain in an affected area in the following manner. That is, magnetic fields are respectively generated at these coils for high-frequency output and low-frequency output via a high-frequency signal and a low-frequency signal with certain frequencies that are outputted from the oscillation circuit. By placing the housing on an affected area of a living body, the affected area will be irradiated with the magnetic fields to have the cells in the affected area stimulated, whereby this stimulation promotes the production of the neurotrophic factors in the cells of the affected area and thereby promotes the repair, growth, differentiation, and proliferation of these cells.

Moreover, Patent Literature 2 discloses a system or the like for electromagnetic induction therapy that is configured as follows. The system includes one or more probes that are ergonomic or contoured to a specific region of the body. This probe includes one or more conductive coils that are configured to generate an electromagnetic field or magnetic field focused on a target nerve, muscle or other body tissues in proximity to the coil. One or more sensors are utilized to detect stimulation and provide feedback about the efficacy of the applied electromagnetic induction therapy. A controller is adjustable to vary a current through the coil so as to adjust the magnetic field focused upon the target nerve, muscle or other body tissues based on the feedback provided by the sensor or the patient.

### Prior art literature

### Patent literature

Patent literature 1: WO2008/056414
Patent literature 2: Japanese Unexamined Patent Application Publication (Translation of PCT Application) No.2013-508119

### Summary of Invention

### Technical Problem

However, the problem with the apparatus disclosed in Patent Literature 1 is that since the coil for high-frequency output and the coil for low-frequency output are received in the housing together with the oscillation circuit and the battery, the housing needs to be placed on an affected area in order for the affected area to be irradiated with the magnetic fields generated at these coils, thereby making the housing bulky and a hindrance, or even causing the apparatus to fall off the affected area after being caught on clothes.

Here, it was made clear that if downsizing the housing in order to solve this problem, the oscillation circuit and the battery need to be downsized as well, which will incur another problem that a sufficient magnetic field intensity and operating time may not be achieved.

The sensor disclosed in Patent Literature 2 is to detect the electric conduction of the nerves stimulated by electromagnetic treatment; this sensor is not configured to directly detect high-frequency magnetic field intensities, probe abnormalities and the like, and a communicative means is not identified as well. Further, mainly, the coil(s) and the sensor are provided separately, and their connections to the control part are also established separately with different wirings. That is, no consideration is given to a problem of accommodating both a communication signal wire between the probe and the main body device and the wiring for high-frequency current supply in one identical signal cable.

In the meantime, as for a magnetic treatment apparatus generating high-frequency signals in such a manner, it is required by, for example, the EN55011 international standard (Industrial, scientific and medical equipment. Radio-frequency disturbance characteristics. Limits and methods of measurement) that the harmonic noise level of a high-frequency signal be reduced so that other devices and equipment will not be subjected to impacts such as malfunction.

However, with the conventional magnetic treatment apparatus, since a high-frequency signal of a single frequency is used in the high-frequency coil, a higher harmonic noise level will be observed if enhancing the output of the high-frequency signal. Thus, it has been difficult for the abovementioned magnetic treatment apparatus to meet the emission standard (EMI standard) detailing the impacts imposed on other apparatuses by the interference waves emitted from the apparatus, and the immunity standard (EMS standard) regulating the performance deterioration and malfunction of the apparatus, while achieving a sufficient therapeutic effect required to treat pain in an affected area.

The present invention was made in view of these technical issues. It is an object of the present invention to provide a magnetic treatment apparatus configured in such a manner where a high-frequency signal is detected by detecting a magnetic field intensity generated from the magnetic treatment apparatus, where monitoring is conducted so that the magnetic field intensity of the magnetism generated by the high-frequency signal will not reach an excessive level while achieving a therapeutic effect required to treat pain in an affected area by increasing the output of the high-frequency signal, and where the output of the high-frequency signal will be immediately stopped once the magnetic field intensity has reached an excessive level, thereby ensuring the safety of the apparatus.

### Solution to Problem

In order to solve the above problems, the magnetic treatment apparatus of the present invention is a magnetic treatment apparatus for treating pain in an affected area of a living body by producing a biostimulation signal wave and stimulating cells and nerves in and around the affected area as a result of irradiating the affected area of the living body with a magnetic field for stimulating the affected area that is generated at a coil via the biostimulation signal wave. This magnetic treatment apparatus includes:
an apparatus main body having a signal wave output part configured to produce and output a biostimulation signal wave of a first frequency; and
a probe formed separately from the apparatus main body and having a first coil that is connected to the signal wave output part through a signal cable and is supplied with the biostimulation signal wave of the first frequency outputted from the signal wave output part, wherein
the first coil is formed as a planar body on one or both surfaces of a flexible sheet, and a coil for magnetic field intensity detection that is adjacent to the first coil is formed on one or both surfaces of the flexible sheet.

Further, with regard to the magnetic treatment apparatus of the present invention, it is considered that more preferred solutions can be brought when, for example,
(a) the coil for magnetic field intensity detection is formed on an outermost edge of the flexible sheet;
(b) the frequency of the biostimulation signal wave of the first frequency varies within a given range with 250 MHz being the center frequency;
(c) the probe further has a magnetic field intensity detection part configured to detect a magnetic field intensity generated at the first coil via the biostimulation signal wave of the first frequency that is detected by the coil for magnetic field intensity detection;
(d) the signal wave output part further produces and outputs a biostimulation signal wave of a second frequency which is different from the first frequency, and the probe further has a second coil that is connected to the signal wave output part through the signal cable and is supplied with the biostimulation signal wave of the second frequency from the signal wave output part; and
(e) the frequency of the biostimulation signal wave of the second frequency is not lower than 1 KHz but not higher than 3 KHz.

### Advantageous Effects of Invention

The magnetic treatment apparatus according to the present invention is such that the signal wave output part of the apparatus main body produces and outputs the biostimulation signal wave of the first frequency, the first coil of the probe formed separately from the apparatus main body is connected to the signal wave output part through the signal cable and is supplied with the biostimulation signal wave of the first frequency outputted from the signal wave generation part, whereby a high-frequency alternating magnetic field for affected area stimulation is generated with such biostimulation signal wave of the first frequency.

Further, in the magnetic treatment apparatus of the present invention, the biostimulation signal wave of the first frequency is detected by the coil for magnetic field intensity detection that is adjacent to the first coil so as to detect and monitor the magnetic field intensity of the magnetic field generated at the first coil. Thus, the magnetic field generated at the first coil can be maintained at a moderate level without causing the magnetic field intensity generated at the first coil to reach an excessive level. As a result, the magnetic treatment apparatus of the present invention is able to ensure its safety by proactively preventing an output runaway caused by the first coil.

Therefore, according to the magnetic treatment apparatus of the present invention, by placing the probe separated from the apparatus main body on an affected area of a living body while proactively preventing the output runaway caused by the first coil, the cells in the affected area will be stimulated as a result of irradiating the affected area with the high-frequency alternating magnetic field that has been generated at the first coil, thereby bringing an expectation that this stimulus will activate the damaged sensory cells in the affected area to induce neurotrophic factors whereby the nerve disorder in the affected area can be alleviated.

Moreover, according to the magnetic treatment apparatus of the present invention, since the first coil in the probe is provided with the coil for magnetic field intensity detection that is adjacent to the first coil, the magnetic field intensity generated at the first coil can be detected by such coil for magnetic field intensity detection. Thus, the magnetic field intensity generated at the first coil can be monitored, and an alarm signal (e.g., sounding of an alarm tone and display of an alarm message) can be outputted by an alarm control part when an abnormality in the magnetic field intensity has been detected. As a result, in the case of the magnetic treatment apparatus of the present invention, the magnetic field intensity will not reach an excessive level; or even when the magnetic field intensity has reached an excessive level, the output of the biostimulation signal wave of the first frequency will be immediately stopped, thereby making it possible to generate a high-frequency alternating magnetic field for affected area stimulation that has a moderate intensity.

Here, in the magnetic treatment apparatus of the present invention, the frequency of the biostimulation signal wave of the first frequency varies within a given range with 250 MHz being the center frequency, preferably within a range of 250 MHz ±20%, more preferably within a range of 250 MHz ±10%. In this way, a high-frequency alternating magnetic field generated via the biostimulation signal wave of the first frequency with 250 MHz being the center has a high effect of inducing neurotrophic factors by activating damaged sensory cells, thereby bringing an expectation that the effect of alleviating the nerve disorder in an affected area can be improved.

Further, the magnetic treatment apparatus of the present invention may also be configured in such a manner where the signal wave output part also produces and outputs the biostimulation signal wave of the second frequency that is different from the first frequency, and the probe also has the second coil that is connected to the signal wave output part through the signal cable and is supplied with the biostimulation signal wave of the second frequency that is outputted from the signal wave output part. If the probe also has such second coil, there may further be provided another coil for magnetic field intensity detection for detecting the magnetic field generated from the second coil. In this way, the stimulus delivered on an affected area as a result of irradiating the affected area with a low-frequency alternating magnetic field for affected area stimulation that is generated at the second coil via the biostimulation signal wave of the second frequency will reach the brain (sensory area) from the spinal cord dorsal horn through sensory nerves (Aβ fibers: sense of touch), thereby allowing the brain to recognize a pleasant sense of touch, and thus also bringing an expectation that a pain-relieving effect and a relaxing effect can be achieved by activating the descending pain inhibitory system.

In addition, in the magnetic treatment apparatus of the present invention, the frequency of the biostimulation signal wave of the second frequency may be not lower than 1 KHz but not higher than 3 KHz. In this way, the stimulus delivered by the low-frequency alternating magnetic field of 1 to 3 KHz that is generated at the second coil via such biostimulation signal wave of the second frequency is particularly easy to be transmitted from the spinal cord dorsal horn to the brain through sensory nerves, thereby bringing an expectation that there can be achieved nerve disorder alleviating effects such as a higher pain-relieving effect and relaxing effect.

### Brief Description of Drawings

[FIG.1] is a perspective view showing the overall appearance of a magnetic treatment apparatus of an embodiment of the present invention.
[FIG.2] is a front view showing the appearance of an apparatus main body of the magnetic treatment apparatus of this embodiment.
[FIG.3] is a set of schematic views showing the appearance of a probe of the magnetic treatment apparatus of this embodiment, in which FIG.3 (a) is a front view, FIG.3(b) is a top view, and FIG.3(c) is a right side surface A-A cross-sectional view.
[FIG.4] is a set of printed wiring diagrams showing a coil arrangement of the magnetic treatment apparatus of this embodiment, in which FIG.4(a) is a top view, and FIG.4(b) is a back view.
[FIG.5] is a block diagram showing the configuration of the magnetic treatment apparatus of this embodiment via functional blocks.
[FIG.6] is a graph showing a magnetic field intensity that is generated from a coil for high-frequency output and is detected by the probe of the magnetic treatment apparatus of this embodiment via a coil for magnetic field intensity detection.

### Description of Embodiments

A magnetic treatment apparatus of this embodiment is described in detail hereunder based on the drawings. Here, each drawing is schematic and may differ from the embodiment in reality. Further, the following embodiment is a set of examples of an apparatus embodying the technical concept of the present invention and is not to limit the configuration of the present invention to those shown below. That is, various modifications can be made to the technical concept of the present invention within the technical scope described in the claims.

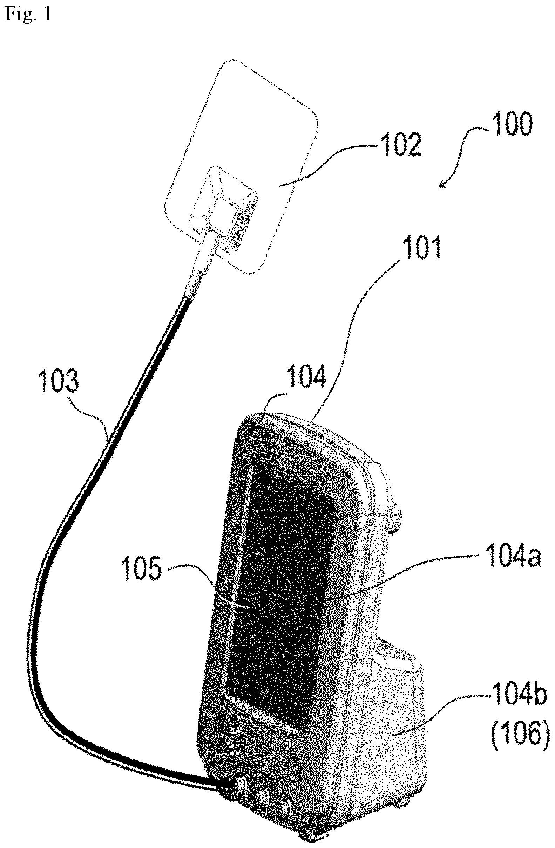

FIG. 1 is a perspective view showing the overall appearance of a magnetic treatment apparatus 100 of an embodiment of the present invention. As shown in FIG. 1, the magnetic treatment apparatus 100 of this embodiment has an apparatus main body 101, a probe 102, a signal cable 103 for connecting the probe 102 to the apparatus main body 101, and a power cable that is not shown and is detachably plugged into and attached to the apparatus main body 101.

The magnetic treatment apparatus 100 of this embodiment is a magnetic treatment apparatus for treating pain in an affected area of a living body by producing biostimulation signal waves and stimulating the cells in the affected area as a result of irradiating the affected area with magnetic fields that are generated at coils via such biostimulation signal waves. The apparatus main body 101 of the magnetic treatment apparatus 100 has a signal wave output part that produces and outputs a biostimulation signal wave of a first frequency. The apparatus main body 101 has a resin casing 104; and a touch input-type (capacitive) display 105 that is housed obliquely upward in the casing 104 and is exposed from an opening section 104a of a front surface of the casing 104. For example, when the display 105 is a color liquid crystal display, the number of display pixels thereof can be set to 1,024 px × 768 px. A battery 106 for supplying a power source required to put the magnetic treatment apparatus 100 into operation is incorporated in a protruding part 104b provided on the rear lower side of the casing 104. Here, the specifications of the battery 106 can be appropriately configured. For example, the battery 106 may be a rechargeable lithium secondary battery whose continuous operating time is set to 8 to 24 hours or longer, and whose charging time is set to within 14 hours.

The magnetic treatment apparatus 100 has the probe 102 formed separately from the apparatus main body and having a first coil that is connected to the signal wave output part through the signal cable 103 and is supplied with the biostimulation signal wave of the first frequency which is outputted from such signal wave output part. The probe 102 is a member for treating pain in an affected area of a living body by generating a magnetic field(s) when placed on the affected area and by irradiating the affected area with such magnetic field at the time of using the magnetic treatment apparatus 100 of this embodiment. It will suffice if the magnetic treatment apparatus 100 has at least one probe 102; the number of the probes 102 may be increased as appropriate. Here, the signal cable 103 may be configured integrally with the apparatus main body 101 and the probe 102, configured in a detachable (inlet) manner with respect to the apparatus main body 101 and the probe 102, or configured separately therefrom. The cable length of the signal cable 103 can be appropriately selected in accordance with the use of the magnetic treatment apparatus 100. For example, the cable length of the signal cable 103 can be set to 0.5 to 2.0 m.

In the magnetic treatment apparatus 100 of this embodiment, the probe 102 is provided as a component separate from the apparatus main body 101. Thus, by placing only the probe 102 on an affected area of a living body, the affected area can be irradiated with, for example, a magnetic field generated at a coil for high-frequency output. Here, a signal wave generation part and a power supply part are not housed in the probe 102. For this reason, the probe 102 can be designed in a more compact fashion as compared to the apparatus main body 101. As a result, when using the magnetic treatment apparatus 100, the probe 102 will not become bulky, get caught on cloths, and then fall off the affected area.

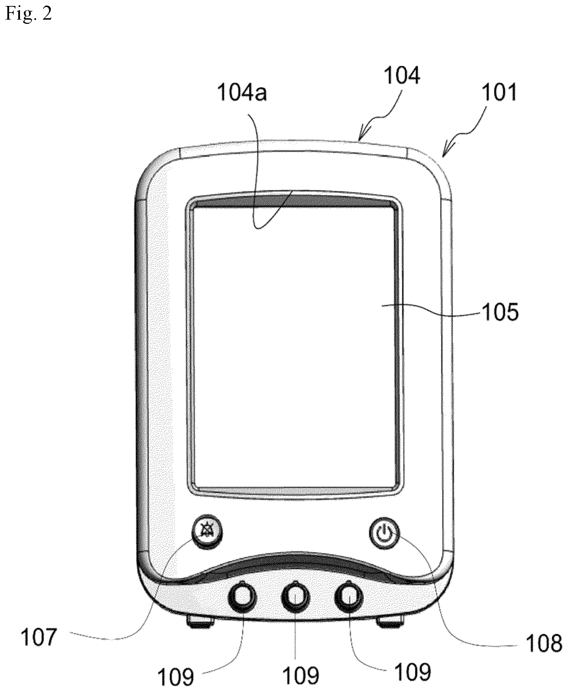

FIG.2 is a front view showing the appearance of the apparatus main body of the magnetic treatment apparatus of this embodiment. As shown in FIG.2, an alarm stop button 107 and a power switch button 108 are provided on the left and the right side below the opening section 104a on the front surface of the casing 104 of the apparatus main body 101. Provided even below the alarm stop button 107 and the power switch button 108 are three horizontally aligned sockets 109 for plugging in the signal cable 103 so that three probes 102 can be connected to the apparatus main body 101. Here, the number of the sockets 109 can be appropriately selected depending on the number of the probes 102 installed.

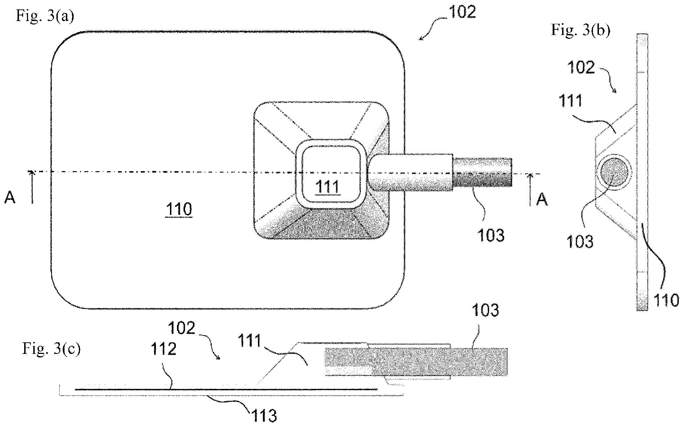

FIG.3 is a set of schematic views showing the appearance of the probe of the magnetic treatment apparatus of this embodiment. FIG.3(a) is a front view of the probe. As shown in FIG.3(a), the probe 102 of the magnetic treatment apparatus 100 of this embodiment has a substantially quadrilateral plate-shaped probe housing 110; and a circuit part 111 for protecting electric circuits or the like formed on a flexible sheet that is received in the plate-shaped probe housing 110. The circuit part 111 is formed in such a manner that it is bulged on the surface of the plate-shaped probe housing 110.

FIG.3(b) is a top view of the probe of the magnetic treatment apparatus, and FIG.3(c) is a right side surface A-A cross-sectional view of the probe of the magnetic treatment apparatus. As shown in FIGs.3(b) and 3(c), the circuit part 111 is formed in such a manner that it is bulged into a truncated pyramid shape toward the surface of the plate-shaped probe housing 110 so that electric circuits or the like formed on a flexible sheet 112 can be protected thereby. The signal cable 103 is connected to the central portion of the circuit part 111. A back surface 113 of the plate-shaped probe housing 110 is a contact surface that comes into contact with an affected area and is an irradiation surface by which the affected area is irradiated with a high-frequency alternating magnetic field for affected area stimulation having a moderate intensity. Here, when the magnetic field intensity has reached an excess level, an alarm signal (e.g., sounding of an alarm tone, display of an alarm message, and vibration alarm) will be outputted by an alarm control part in the magnetic treatment apparatus.

For the sake of maintaining the flexibility of the probe 102, it is preferred that the plate-shaped probe housing 110 be formed of a flexible material. The plate-shaped probe housing 110 is preferably formed of a soft resin that is superior in, for example, water resistance and heat resistance, and does not affect the human body even when in contact with the skin as an affected area. There are no particular limitations on the material for forming the plate-shaped probe housing 110 as long as the aforementioned properties are satisfied; examples of such material may include a silicon resin, an elastomer, and a rubber material. The plate-shaped probe housing 110 can be produced via resin molding such as injection molding and RIM molding. Here, the plate-shaped probe housing 110 preferably has a shape that allows the flexible sheet 112 to be received therein and is substantially identical to a first coil 102a formed on the flexible sheet 112.

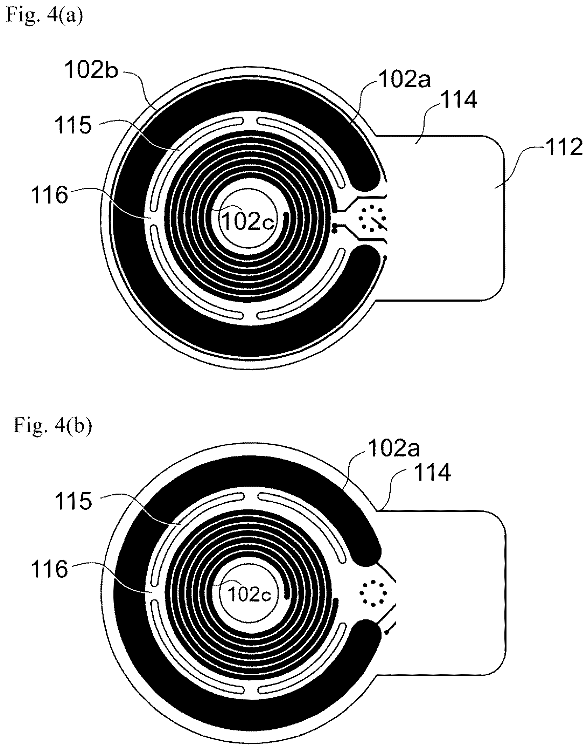

FIG.4 is a set of printed wiring diagrams showing a coil arrangement of the magnetic treatment apparatus of this embodiment. As shown in FIG.4, the probe 102 of the magnetic treatment apparatus 100 of this embodiment has the first coil 102a supplied with the biostimulation signal wave of the first frequency that is outputted from the signal wave output part of the apparatus main body 101. The first coil 102a is formed as a planar body on one or both surfaces of the flexible sheet 112 received in the probe 102. Further, a coil for magnetic field intensity detection 102b adjacent to the first coil 102a is formed on one or both surfaces of the flexible sheet 112. That is, the probe 102 of the magnetic treatment apparatus 100 of this embodiment is such that since there is provided the coil for magnetic field intensity detection 102b that is adjacent to the first coil 102a, a high-frequency alternating magnetic field for affected area stimulation which is a magnetic field generated from the first coil 102a can be constantly detected by detecting the biostimulation signal wave of the first frequency that is outputted by the first coil 102a. In other words, the magnetic treatment apparatus 100 of this embodiment is such that since there are provided the first coil 102a that is received inside the probe 102 and the coil for magnetic field intensity detection 102b that is adjacent to such coil for high-frequency output, not only a therapeutic effect required to treat pain in an affected area can be achieved by increasing the output of a high-frequency signal, but the safety of the magnetic treatment apparatus itself can be ensured by performing detection so that an excess high-frequency alternating magnetic field will not be generated.

The first coil 102a may be formed on both the front and back surfaces of the flexible sheet 112. Further, the first coil 102a may be formed only on one of the front and back surfaces of the flexible sheet 112. As for the coil for magnetic field intensity detection 102b, it will suffice if the coil for magnetic field intensity detection 102b is formed adjacent to the first coil 102a; the coil for magnetic field intensity detection 102b may be formed on the outermost edge of the flexible sheet 112 or inside the first coil 102a.

FIG.4(a) is a top view of the printed wiring diagrams. As shown in FIG.4(a), arranged on the front surface of the flexible sheet 112 are an electric circuit 114, the first coil 102a, the coil for magnetic field intensity detection 102b, a second coil 102c and the like. That is, in this embodiment, the first coil 102a and the coil for magnetic field intensity detection 102b are arranged on the upper surface (front surface) of the flexible sheet 112 in such a manner that the first coil 102a is provided as an annular disk, and the coil for magnetic field intensity detection 102b that has an annular shape is provided adjacent to and outside such first coil 102a.

Further, the second coil 102c that has a spiral shape is arranged inside the first coil 102a. In the electric circuit 114 arranged on the flexible sheet 112, there are formed, for example, a circuit for detecting the magnetic field generated from the high frequency detected by the coil for magnetic field intensity detection 102b, and a circuit for detecting an operation state via a temperature detection element and a CPU.

The first coil 102a generates the high-frequency alternating magnetic field for affected area stimulation via the biostimulation signal wave of the first frequency that is outputted from the signal wave output part of the apparatus main body 101 and is supplied through the signal cable 103. The second coil 102c generates a low-frequency alternating magnetic field for affected area stimulation via a biostimulation signal wave of a second frequency that is outputted from the signal wave output part of the apparatus main body 101 and is supplied through the signal cable 103.

That is, on the front surface of the flexible sheet 112 received in the plate-shaped probe housing 110, there are formed, via printed wiring, the first coil 102a that is positioned in an outward region on the front surface of the flexible sheet 112; the second coil 102c that is positioned inside such first coil 102a; and the coil for magnetic field intensity detection 102b that is positioned outside and adjacent to the first coil 102a. In such printed wiring shown in FIG.4(a), the coil for magnetic field intensity detection 102b adjacent to the first coil 102a is formed on the outermost edge of the flexible sheet 112. With the coil for magnetic field intensity detection 102b being arranged adjacent to the first coil 102a, the intensity of the magnetic field generated from the first coil 102a can be detected with a high sensitivity. It is preferable if the coil for magnetic field intensity detection 102b is formed inside the first coil 102a, because a higher detection sensitivity of the magnetic field intensity can be achieved. Meanwhile, it is preferable if the coil for magnetic field intensity detection 102b is formed outside the first coil 102a, because the coil for magnetic field intensity detection 102b is less likely to be affected by the second coil 102c. In addition, this is preferable because a higher detection sensitivity of the magnetic field intensity can be achieved.

FIG.4(b) is a back view of the printed wiring diagrams. As shown in FIG.4(b), in the magnetic treatment apparatus 100 of this embodiment, other than the front surface of the flexible sheet 112, the first coil 102a and the second coil 102c may also be formed on the back surface of the flexible sheet 112. In this way, the front and back surfaces of the flexible sheet 112 may be configured in a plane-symmetrical manner and arranged in such a way that, for example, a high-frequency current flowing in the first coil 102a and a low-frequency current flowing in the second coil 102c respectively flow in the same direction on both the front and back surfaces. Thus, the centers of the magnetic fields generated by the first coils 102a and the second coils 102c that are arranged on both the front and back surfaces of the flexible sheet 112 can be coincident with each other.

Moreover, as shown in FIGs. 4(a) and 4(b), slit sections 115 are provided between the first coil 102a and the second coil 102c of the flexible sheet 112, whereby a followability of the flexible sheet 112 to the probe 102 is improved upon deformation of the probe 102. Further, multiple parts on the circumference of the slit sections 115 are connected to one another through bridge portions 116, which makes it possible to stably carry out molding when covering the flexible sheet 112 via resin molding. Here, instead of an annular disk, the first coil 102a may also be substantially formed into a rectangular shape.

The flexible sheet 112 is consisted of a film-shaped printed board preferably having a base layer that is composed of a film made of a bendable insulating material; and, if necessary, an adhesive layer. The base layer may employ, for example, a polyimide resin, a polyester resin, a polyamide paper substrate epoxy resin, a glass cloth substrate epoxy resin, and a glass substrate BT resin. The first coil 102a, the second coil 102c, and the coil for magnetic field intensity detection 102b may each be a conductor printed or etched on the flexible sheet 112, such as a metal foil.

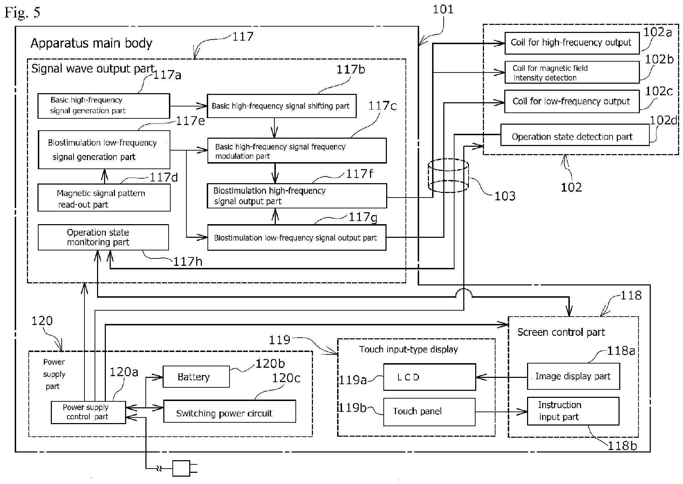

FIG.5 is a block diagram showing the configuration of the magnetic treatment apparatus of this embodiment via functional blocks. As shown in FIG.5, the magnetic treatment apparatus 100 of this embodiment has the apparatus main body 101, the probe 102, and the signal cable 103. From a functional perspective, the apparatus main body 101 has a signal wave output part 117, a screen control part 118, a touch input-type display 119, and a power supply part 120.

The circuit configuration of the signal wave output part 117 in this embodiment is such that it is configured using a plurality of central processing units (CPU) that are not shown and are provided on a printed-wiring board(s) installed inside the apparatus main body 101. Here, FIG.5 illustrates an example where the biostimulation signal wave of the first frequency is a biostimulation high-frequency signal, and the biostimulation signal wave of the second frequency is a biostimulation low-frequency signal. The signal wave output part 117 has a basic high-frequency signal generation part 117a, a basic high-frequency signal shifting part 117b, a basic high-frequency signal frequency modulation part 117c, and a biostimulation high-frequency signal output part 117f that are configured to output the biostimulation high-frequency signal. The basic high-frequency signal generation part 117a generates, for example, a basic high-frequency signal of 250 MHz. The basic high-frequency signal shifting part 117b moderately shifts (varies) the basic high-frequency signal generated by the basic high-frequency signal generation part 117a. For example, the basic high-frequency signal shifting part 117b moderately shifts (varies) the basic high-frequency signal every fixed amount of time of, for example, 0.00014 seconds, with 250 MHz being the center frequency and within a range of 225 MHz to 275 MHz which is ±10% from such center frequency. Here, when the basic high-frequency signal shifting part 117b moderately shifts (varies) the basic high-frequency signal, the frequency band used in emergency locator transmitter for an airplane is excluded. The basic high-frequency signal shifting part 117b outputs the moderately shifted (varied) basic high-frequency signal to the basic high-frequency signal frequency modulation part 117c.

The signal wave output part 117 is also configured as follows. A magnetic signal pattern read-out part 117d reads out magnetic signal patterns (e.g., sound source signals such as pleasant music) from a recording medium such as an SD card, and then supplies them to a biostimulation low-frequency signal generation part 117e, the magnetic signal patterns being previously recorded in a recording medium such as an SD card inserted in a card slot on a printed-wiring board built in the apparatus main body 101. The biostimulation low-frequency signal generation part 117e processes the magnetic signal patterns (e.g., sound source signals such as pleasant music) with a bandpass filter or the like, generates a biostimulation low-frequency signal based on the frequency information (e.g., 1 to 3 KHz) of the magnetic signal patterns, and outputs such biostimulation low-frequency signal to the basic high-frequency signal frequency modulation part 117c and a biostimulation high-frequency signal output part 117f.

Using the biostimulation low-frequency signal generated by the biostimulation low-frequency signal generation part 117e, the basic high-frequency signal frequency modulation part 117c modulates the frequency of a basic high-frequency signal of, for example, 250 MHz ±10% that has been obtained by shifting (varying), via the basic high-frequency signal shifting part 117b, the frequency of the basic high-frequency signal generated by the basic high-frequency signal generation part 117a. The basic high-frequency signal frequency modulation part 117c supplies a biostimulation high-frequency signal to the biostimulation high-frequency signal output part 117f, the biostimulation high-frequency signal being generated by modulating the frequency of the frequency-shifted (varied) basic high-frequency signal with the biostimulation low-frequency signal. The biostimulation high-frequency signal output part 117f amplifies such frequency-modulated biostimulation high-frequency signal and then outputs the amplified signal to the coil for high-frequency output 102a of the probe 102. Here, the biostimulation high-frequency signal output part 117f may also modulate the amplitude of such frequency-modulated biostimulation high-frequency signal with the biostimulation low-frequency signal before amplifying the high-frequency signal and then outputting the amplified signal to the coil for high-frequency output 102a of the probe 102.

Further, a biostimulation low-frequency signal output part 117g amplifies, for example, a biostimulation low-frequency signal of not lower than 1 KHz but not higher than 3 KHz that has been generated by the biostimulation low-frequency signal generation part 117e, and outputs the amplified signal to the coil for low-frequency output 102c of the probe 102. These operations at the signal wave output part 117 are monitored by an operation state monitoring part 117h.

The probe 102 in this embodiment is configured in such a manner that the flexible sheet 112 not shown is housed in the plate-shaped probe housing 110 made of a soft resin such as a silicon resin. Formed via printed wiring on the substrate of the flexible sheet 112 are the coil for high-frequency output 102a that is positioned in the outward region; the coil for low-frequency output 102c that is positioned inside such coil for high-frequency output 102a; and the coil for high frequency detection 102b as the coil for magnetic field intensity detection that is positioned outside the coil for high-frequency output 102a. Further, a circuit configured is an operation state detection part 102d using a temperature detection element and a CPU that are mounted on such flexible sheet 112. The coil for high-frequency output 102a generates a high-frequency alternating magnetic field for affected area stimulation with the biostimulation high-frequency signal that is supplied from the biostimulation high-frequency signal output part 117f through the signal cable 103. The coil for low-frequency output 102c generates a low-frequency alternating magnetic field for affected area stimulation with the biostimulation low-frequency signal that is supplied from the biostimulation low-frequency signal output part 117g through the signal cable 103.

The operation state detection part 102d detects the operation state of the signal wave output part 117 from the temperatures of the coil for high-frequency output 102a and the coil for low-frequency output 102c that are detected by the temperature detection element, and from the magnetic field intensity that is detected by the coil for magnetic field intensity detection 102b. The operation state detection part 102d sends a signal indicating such operation state as feedback to the operation state monitoring part 117h of the signal wave output part 117 through the signal cable 103. With this signal indicating the operation state, the operation state monitoring part 117h is able to monitor operations such as the generation and output of signal waves by the signal wave output part 117 as well as whether or not the alternating magnetic fields generated by the coil for high-frequency output 102a and the coil for low-frequency output 102c exhibit excessive magnetic field intensities, and output an alarm signal (e.g., sounding of an alarm, display of an alarm message, and vibration alarm) via an alarm control part (not shown) if detecting an abnormality.

The circuit configuration of the screen control part 118 is such that, on a printed-wiring board installed in the apparatus main body 101, the screen control part is mainly configured using a graphics processing unit (GPU) or the like that is not shown. An image display part 118a of the screen control part 118 reads out screen information such as instruction buttons that are to be displayed on a liquid crystal display (LCD) 119a of the touch input-type display 119. Screen information such as the instruction buttons is previously recorded in an SD card inserted in a card slot on the printed-wiring board. Further, the image display part 118a displays screen information such as the instruction buttons on the liquid crystal display (LCD) 119a.

The image display part 118a detects a position at which the user of the magnetic treatment apparatus has touched a touch panel 119b of the touch input-type display 119 via an instruction input part 118b, based on, for example, changes in static electricity at such position. The image display part 118a sends to the operation state monitoring part 117h a signal indicating the instruction input directed by the operation button displayed on the liquid crystal display (LCD) 119a in response to such touch position. This signal indicating the instruction input allows the operation state monitoring part 117h to detect and monitor, for example, operations such as the generation and output of signal waves by the signal wave output part 117 as well as the magnetic field intensities of the alternating magnetic fields generated by the coil for high-frequency output 102a and the coil for low-frequency output 102c, in accordance with the instructions of the user.

The screen control part 118 creates a log in which recorded are the instruction inputs to the instruction input part 118b by the operation buttons displayed on the liquid crystal display (LCD) 119a, the operation state of the signal wave output part 117 at that time, and the magnetic field intensity of the magnetic field generated from the high-frequency signal. The log information created by the screen control part 118 is stored in a USB memory slot that is disposed between two pieces of printed-wiring board installed in the casing protruding part 104b on the rear lower side of the casing 104 and is covered by an openable and closable lid. The USB memory slot may be installed in such a manner that it is able to be inserted into and removed from an upper surface side of the casing protruding part 104b. Further, the image display part 118a has a clock function for displaying a clock on the liquid crystal display (LCD) 119a. This clock function is maintained by button cells placed in a battery holder on the printed-wiring board installed inside the apparatus main body 101.

The circuit configuration of the power supply part 120 is such that it is configured using a CPU that is not shown and is mounted on the printed-wiring board installed in the apparatus main body 101, two AC-DC converters that are not shown, and the printed-wiring boards provided in the casing protruding part 104b on the rear lower side of the casing 104. The power supply part 120 has a power supply control part 120a on the printed-wiring board. The two pieces of printed-wiring board installed in the apparatus main body 101 are each supplied with a commercial alternating-current power source of 36V supplied through a power cable (not shown) that is detachably plugged into and attached to a power socket disposed rearward on the casing protruding part 104b on the rear lower side of the casing 104. A direct-current power source of 24V is obtained with a switching power circuit 120c on one printed-wiring board installed in the apparatus main body 101. Further, a direct-current power source of 12V is obtained with a switching power circuit on the other printed-wiring board installed in the apparatus main body 101. By connecting these direct-current power sources in series, a battery 120b of 36V is charged.

During a normal use of the magnetic treatment apparatus to which the power cable is not attached, the power supply part 120 supplies the direct-current power source of 36V from the battery 120b to the signal wave output part 117 and screen control part 118 of the apparatus main body 101 and to the operation state detection part 102d of the probe 102 after stepping down the voltage to direct-current voltages required for each part via the two AC-DC converters, thereby allowing this magnetic treatment apparatus to be used in a portable manner.

In the case of the magnetic treatment apparatus of this embodiment, the biostimulation high-frequency signal is generated by the basic high-frequency signal generation part 117a, basic high-frequency signal shifting part 117b, and basic high-frequency signal frequency modulation part 117c of the signal wave output part 117 of the apparatus main body 101. The biostimulation high-frequency signal output part 117f outputs the biostimulation high-frequency signal to the coil for high-frequency output 102a of the probe 102. The coil for high-frequency output 102a of the probe 102 that is configured separately from the apparatus main body 101 generates the high-frequency alternating magnetic field for affected area stimulation via the biostimulation high-frequency signal supplied from the biostimulation high-frequency signal output part 117f. Here, the biostimulation high-frequency signal is, for example, a high-frequency signal whose center frequency is 250 MHz.

Thus, according to the magnetic treatment apparatus of this embodiment, by allowing the coil for magnetic field intensity detection 102b to detect the high frequency generated at the coil for high-frequency output 102a, monitoring is conducted so that the high-frequency alternating magnetic field does not reach an excessive magnetic field intensity, and an affected area can then be irradiated with a moderate high-frequency alternating magnetic field so as to have the cells in the affected area stimulated, thereby bringing an expectation that this stimulus will activate, for example, the damaged sensory cells in the affected area to induce neurotrophic factors whereby the nerve disorder in the affected area is alleviated. In addition, the high-frequency alternating magnetic field generated from the coil for high-frequency output 102a, which is generated from a high frequency with a center of 250 MHz, has a high effect of inducing neurotrophic factors by activating damaged sensory cells, thereby bringing an expectation that the effect of alleviating the nerve disorder in the affected area can be improved.

Further, according to the magnetic treatment apparatus of this embodiment, the basic high-frequency signal shifting part 117b of the signal wave output part 117 in the apparatus main body 101 varies the frequency of the biostimulation high-frequency signal within a range of, for example, 250 MHz ±10%, and the biostimulation high-frequency signal is detected by the coil for magnetic field intensity detection 102b, thereby making it possible to perform monitoring so that the magnetic field generated at the coil for high-frequency output can be maintained at a moderate level without causing the magnetic field intensity of the magnetic field generated from the biostimulation high-frequency signal to reach an excessive level. As a result, the magnetic treatment apparatus of the present invention is capable of ensuring its safety by proactively preventing an output runaway caused by the coil for high-frequency output, and is capable of generating a high-frequency alternating magnetic field for affected area stimulation with a high strength by supplying to the high-frequency coil a biostimulation high-frequency signal having a high strength that is within the EMI standard.

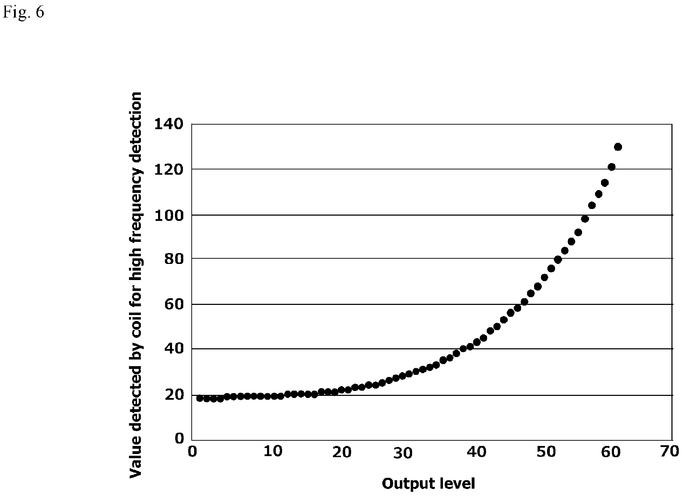

FIG.6 is a graph showing, as a working example, a correlation between magnetic field intensity and an output signal level of the biostimulation high-frequency signal output part 117f when the probe of the magnetic treatment apparatus of this embodiment is equipped with the coil for magnetic field intensity detection. In the graph shown in FIG.6, the horizontal axis of the graph indicates amplitude modulation data of a high frequency in 64 stages; the vertical axis of the graph indicates values of magnetic field intensities detected by the coil for magnetic field intensity detection (high frequency detection coil) as the output level was raised from 0.

This result shows that with the magnetic treatment apparatus of this embodiment, the basic high-frequency signal shifting part 117b of the signal wave output part 117 in the apparatus main body 101 varies the frequency of the biostimulation high-frequency signal within the given range, and the biostimulation high-frequency signal is detected by the coil for magnetic field intensity detection 102b of the probe 102, thereby proactively preventing the magnetic field intensity of the magnetic field generated from the biostimulation high-frequency signal from reaching an excessive level, thus allowing the magnetic field generated at the coil for high-frequency output to be maintained at a moderate level. It was proven that the magnetic treatment apparatus of this embodiment was capable of proactively preventing an output runaway caused by the coil for high-frequency output, and was capable of generating a high-frequency alternating magnetic field for affected area stimulation with a moderate magnetic field intensity by supplying to the high-frequency coil a biostimulation high-frequency signal having a high strength that is within the EMI standard.

Further, according to the magnetic treatment apparatus of this embodiment, the biostimulation low-frequency signal generation part 117e of the signal wave output part 117 in the apparatus main body 101 produces the biostimulation low-frequency signal from the magnetic signal patterns. The biostimulation low-frequency signal output part 117g outputs such biostimulation low-frequency signal to the coil for low-frequency output 102c of the probe 102. The coil for low-frequency output 102c of the probe 102 is connected to the biostimulation low-frequency signal output part 117g of the signal wave output part 117 through the signal cable 103 and is supplied with the biostimulation low-frequency signal. Thus, the stimulus delivered on an affected area as a result of irradiating the affected area with the low-frequency alternating magnetic field for affected area stimulation that is generated at the coil for low-frequency output 102c via the biostimulation low-frequency signal will reach the brain (sensory area) from the spinal cord dorsal horn through sensory nerves (Aβ fibers: sense of touch), thereby allowing the brain to recognize a pleasant sense of touch, and thus also bringing an expectation that a pain-relieving effect and a relaxing effect can be achieved by activating the descending pain inhibitory system.

Moreover, according to the magnetic treatment apparatus of this embodiment, the basic high-frequency signal frequency modulation part 117c of the signal wave output part 117 in the apparatus main body 101 produces the biostimulation high-frequency signal by modulating the frequency of the basic high-frequency signal with the biostimulation low-frequency signal produced by the signal wave output part 117, and the biostimulation high-frequency signal output part 117f supplies such frequency-modulated biostimulation high-frequency signal to the coil for high-frequency output 102a of the probe 102. Further, the biostimulation high-frequency signal supplied to the coil for high-frequency output 102a of the probe 102 is detected by the coil for magnetic field intensity detection 102b of the probe 102. In this way, monitoring is conducted so that an excessive magnetic field intensity will not be exhibited by the high-frequency alternating magnetic field for affected area stimulation that has been generated at the coil for high-frequency output 102a via the biostimulation high-frequency signal that has been frequency-modulated with the biostimulation low-frequency signal. Thus, by stimulating the cells in an affected area with such moderate high-frequency alternating magnetic field, the damaged sensory cells in the affected area are more activated as compared to when no frequency modulation is conducted, thereby inducing more neurotrophic factors, thus bringing an expectation that the nerve disorder in the affected area can be more alleviated.

In addition, according to the magnetic treatment apparatus of this embodiment, the signal wave output part 117 of the apparatus main body 101 also produces and outputs the biostimulation low-frequency signal, and the probe 102 is connected to the signal wave output part 117 through the signal cable 103. The probe 102 also has the coil for low-frequency output 102c to which the biostimulation low-frequency signal is supplied from the signal wave output part 117. The signal wave output part 117 produces the biostimulation high-frequency signal by modulating the frequency of the basic high-frequency signal with the biostimulation low-frequency signal, and outputs the biostimulation high-frequency signal separately from the biostimulation low-frequency signal. Thus, with the magnetic treatment apparatus of this embodiment, not only there can be brought about a pain-relieving effect and a relaxing effect with the low-frequency alternating magnetic field for affected area stimulation that is generated at the coil for low-frequency output 102c via the biostimulation low-frequency signal, but there is also an expectation that the nerve disorder in an affected area can be more alleviated with a moderate high-frequency alternating magnetic field for affected area stimulation whose magnetic field intensity is constantly detected in a way such that detected by the coil for magnetic field intensity detection 102b is the high frequency that is generated at the coil for high-frequency output 102a via the biostimulation high-frequency signal obtained by modulating the frequency of the basic high-frequency signal with the biostimulation low-frequency signal.

Further, according to the magnetic treatment apparatus of this embodiment, the frequency of the biostimulation low-frequency signal is not lower than 1 KHz but not higher than 3 KHz, and the stimulus delivered by the low-frequency alternating magnetic field of 1 to 3 KHz that is generated at the coil for low-frequency output 102c via such biostimulation low-frequency signal is particularly easy to be transmitted from the spinal cord dorsal horn to the brain through sensory nerves, thereby bringing an expectation that there can be achieved nerve disorder alleviating effects such as a higher pain-relieving effect and relaxing effect.

The embodiment shown in the drawings has been described as above. However, the magnetic treatment apparatus of the present invention shall not be limited to the above embodiment and may be appropriately modified within the scope of the descriptions in the claims; for example, the basic high-frequency signal shifting part 117b may be configured to shift the basic high-frequency signal of 250 MHz within a range of, for example, 250 MHz ±20%.

Moreover, for example, the basic high-frequency signal frequency modulation part 117c may be configured to produce a biostimulation low-frequency signal having a frequency range of, for example, 1 KHz to 3 KHz with 1.6 KHz being the center frequency, other than the frequency range of 1 to 3 KHz.

### Industrial Applicability

As such, according to the magnetic treatment apparatus of the present invention, the signal wave output part of the apparatus main body produces and outputs the biostimulation signal wave of the first frequency, the first coil (coil for high-frequency output) of the probe formed separately from the apparatus main body is connected to the signal wave output part through the signal cable and is supplied with the biostimulation signal wave of the first frequency that is outputted from the signal wave generation part, whereby there can be generated the high-frequency alternating magnetic field for affected area stimulation via the biostimulation signal wave of the first frequency. Therefore, by placing the probe that is configured separately from the apparatus main body on an affected area of a living body, the cells in the affected area will be stimulated as a result of irradiating the affected area with the high-frequency alternating magnetic field that has been generated at the first coil, thereby bringing an expectation that this stimulus will activate, for example, the damaged sensory cells in the affected area to induce neurotrophic factors whereby the nerve disorder in the affected area can be alleviated.

In addition, according to the magnetic treatment apparatus of this invention, the signal wave output part of the apparatus main body detects the frequency of the biostimulation signal wave of the first frequency (biostimulation high-frequency signal) via the coil for magnetic field intensity detection that is built in the probe, thereby making it possible to detect the magnetic field intensity of the magnetic field generated from the first coil (coil for high-frequency output). Thus, the magnetic treatment apparatus of the present invention is industrially useful, because the safety of the apparatus can be ensured as an output runaway caused by the first coil (coil for high-frequency output) can be proactively prevented without yielding an excessive magnetic field intensity of the magnetic field generated at the first coil (coil for high-frequency output).

The invention of this application has been described as above with reference to an embodiment; however, the invention of this application shall not be limited to the abovementioned embodiment. The configuration and particulars of the invention of this application may be subjected to various modifications that are comprehensible to those skilled in the art within the technical scope of the invention of this application.

### Description of symbols

100 Magnetic treatment apparatus
101 Apparatus main body
102 Probe
102a Coil for high-frequency output (first coil)
102b Coil for high frequency detection (coil for magnetic field intensity detection)
102c Coil for low-frequency output (second coil)
102d Operation state detection part
103 Signal cable
104 Casing
104a Casing opening section
104b Casing protruding part
105 Display
106 Battery
107 Alarm stop button
108 Power switch button
109 Plug socket
110 Plate-shaped probe housing
111 Circuit part (circuit receiving part)
112 Flexible sheet
113 Back surface (probe housing)
114 Electric circuit
115 Slit section
116 Bridge portion
117 Signal wave output part
117a Basic high-frequency signal generation part
117b Basic high-frequency signal shifting part
117c Basic high-frequency signal frequency modulation part
117d Magnetic signal pattern read-out part
117e Biostimulation low-frequency signal generation part
117f Biostimulation high-frequency signal output part
117g Biostimulation low-frequency signal output part
117h Operation state monitoring part
118 Screen control part
118a Image display part
118b Instruction input part
119 Touch input-type display
119a Liquid crystal display (LCD)
119b Touch panel
120 Power supply part
120a Power supply control part
120b Battery
120c Switching power circuit

## Claims

1. A magnetic treatment apparatus for treating pain in an affected area of a living body by producing a biostimulation signal wave and stimulating cells and nerves in and around the affected area as a result of irradiating the affected area with a magnetic field for affected area stimulation that is generated at a coil via the biostimulation signal wave, comprising:
an apparatus main body having a signal wave output part configured to produce and output a biostimulation signal wave of a first frequency; and
a probe formed separately from the apparatus main body and having a first coil that is connected to the signal wave output part through a signal cable and is supplied with the biostimulation signal wave of the first frequency outputted from the signal wave output part,
**characterized in that**
the first coil is formed as a planar body on one or both surfaces of a flexible sheet, and a coil for magnetic field intensity detection that is adjacent to the first coil is formed on one or both surfaces of the flexible sheet.

2. The magnetic treatment apparatus according to claim 1, wherein the coil for magnetic field intensity detection is formed on an outermost edge of the flexible sheet.

3. The magnetic treatment apparatus according to claim 1 or 2, wherein the frequency of the biostimulation signal wave of the first frequency varies within a given range with 250 MHz being the center frequency.

4. The magnetic treatment apparatus according to any one of claims 1 to 3, wherein the probe further has a magnetic field intensity detection part configured to detect a magnetic field intensity generated at the first coil via the biostimulation signal wave of the first frequency that is detected by the coil for magnetic field intensity detection.

5. The magnetic treatment apparatus according to any one of claims 1 to 4, wherein the signal wave output part further produces and outputs a biostimulation signal wave of a second frequency which is different from the first frequency, and the probe further has a second coil that is connected to the signal wave output part through the signal cable and is supplied with the biostimulation signal wave of the second frequency from the signal wave output part.

6. The magnetic treatment apparatus according to claim 5, wherein the frequency of the biostimulation signal wave of the second frequency is not lower than 1 KHz but not higher than 3 KHz.
